# EUROPÄISCHE PATENTSCHRIFT

(11) **EP 1 133 311 B1**
(45) Veröffentlichungstag und Bekanntmachung des Hinweises auf die Patenterteilung: **11.10.2006**
(21) Anmeldenummer: 99960927.4
(22) Anmeldetag: 16.11.1999
(51) Int. Cl.: A61K 38/17, A61K 39/395

(54) **BEEINFLUSSUNG DER ANGIOGENESE DURCH CD66a**
INFLUENCING ANGIOGENESIS USING CD66a
INFLUENCE SUR L'ANGIOGENESE PAR LA CD66a

(30) Priorität: 16.11.1998 DE 19852804
(43) Veröffentlichungstag der Anmeldung: 19.09.2001
(73) Patentinhaber: Wagener, Christoph, 20251 Hamburg (DE)
(72) Erfinder: Wagener, Christoph, 20251 Hamburg (DE); Ergün, Süleyman, 20251 Hamburg (DE)
(74) Vertreter: Schüssler, Andrea
(86) Internationale Anmeldenummer: PCT/DE1999/003671
(87) Internationale Veröffentlichungsnummer: WO 2000/029015

(56) Entgegenhaltungen:
- WO-A-97/00954
- STOFFEL, ARCHONTOULA ET AL: "Monoclonal, anti-domain and anti-peptide antibodies assign the molecular weight 160,000 granulocyte membrane antigen of the CD66 cluster to a mRNA species encoded by the biliary glycoprotein gene, a member of the carcinoembryonic antigen gene family" J. IMMUNOL. (1993), 150(11), 4978-84 , XP000919461 in der Anmeldung erwähnt
- DRABEROVA L ET AL: "A novel monoclonal antibody specific for biliary glycoprotein ( CD66a )." FOLIA BIOLOGICA, (1997) 43 (6) 243-4. , XP000918084
- HUANG J ET AL: "Expression of biliary glycoprotein ( CD66a ) in normal and malignant breast epithelial cells." ANTICANCER RESEARCH, (1998 SEP-OCT) 18 (5A) 3203-12. , XP000918015
- BAMBERGER A M ET AL: "Dysregulated expression of CD66a (BGP, C-CAM), an adhesion molecule of the CEA family, in endometrial cancer." AMERICAN JOURNAL OF PATHOLOGY, (1998 JUN) 152 (6) 1401-6. , XP000918049
- ERGUN S ET AL: "CEA-related cell adhesion molecule 1: a potent angiogenic factor and a major effector of vascular endothelial growth factor." MOLECULAR CELL, (2000 FEB) 5 (2) 311-20. , XP000918037

## Beschreibung

Die Erfindung betrifft eine pharmazeutische Zusammensetzung zur Beeinflussung der Angiogenese. Im einen Fall kann durch Verabreichung von CD66a oder Substanzen, die die Bildung von CD66a initiieren, die Angiogenese gefördert werden, während im anderen Fall durch die Verwendung von Substanzen, die die Interaktion zwischen CD66a und CD66a-Liganden verhindern, die Angiogenese gehemmt werden kann.

Die Ausbildung von Blutgefäßen (Angiogenese) ist für viele Krankheiten ein wichtiger Schritt, der einerseits zur Heilung beitragen kann, aber auch in anderen Fällen wünschenswerterweise unterbunden werden soll. Eine Förderung der Angiogenese ist beispielsweise für Herz-Kreislauf-Krankheiten, z.B. zur Behandlung von Angina pectoris oder Herz- bzw. Hirninfarkten, sehr wünschenswert. Andererseits ist die Hemmung der Gefäßversorgung maligner solider Tumore bei Mensch und Tier ein vielversprechender Ansatz in der Tumortherapie. Angiogeneseinhibitoren, wie beispielsweise Endostatin, greifen direkt normale und daher genetisch stabile Endothelzellen der Blutgefäße, die einen Tumor versorgen, an, bringen diese zum Absterben und unterbinden somit die Versorgung der Tumorzellen mit nährstoffhaltigem Blut (vgl. Kerbel, R., Nature, 390, S. 335ff., 1997). Dadurch kommt es zu einer Regression von Blutgefäßen und Turmormasse. Da die Endothelzellen im Gegensatz zu den Tumorzellen genetisch stabil sind, kommt es nicht zur Ausbildung von Resistenzen, wie es beispielsweise bei der direkt gegen die Tumorzellen gerichteten Cytostatikatherapie der Fall ist. Durch Hemmung der Angiogenese ließ sich in experimentellen Modellen das Wachstum menschlicher Tumore blockieren. Inzwischen befinden sich einige Angiogenesehemmer in der klinischen Erprobung (vgl. Hanahan et al., Cell 86, 353-364 (1996)).

Die Versorgung von Geweben mit neuen Gefäßen ist ein komplexer Prozeß, an dem eine Vielzahl von Biomolekülen beteiligt ist. Tumore produzieren beispielsweise lösliche Mediatoren, die die Ausbildung neuer Gefäße initiieren. Im weiteren Verlauf der Angiogenese spielen Adhäsionsmoleküle eine zentrale Rolle, die die Kommunikation von Gefäßzellen untereinander und mit dem umgebenden Bindegewebe steuern. Schließlich sind an der Neubildung von Gefäßen auch verschiedene Proteinasen beteiligt.

Der vorliegenden Erfindung liegt die Aufgabe zugrunde, eine Möglichkeit bereitzustellen, die Angiogenese je nach Bedarf fördern oder hemmen zu können. Im Fall der Hemmung der Angiogenese soll damit eine Form der Krebstherapie ohne Resistenzentwicklung bereitgestellt werden, d. h. es soll insbesondere in die einen Tumor begleitende Angiogenese im Sinne einer Reduktion bzw. Hemmung eingegriffen werden.

Erfindungsgemäß wird diese Aufgabe durch die Gegenstände der Patentansprüche gelöst.

Gegenstand der vorliegenden Anmeldung ist insbesondere die Verwendung bestimmter Stoffe zur Herstellung einer pharmazeutischen Zusammensetzung, die sich zur Regulierung der Angiogenese eignet. Die Stoffe umfassen:
(a) zur positiven Regulierung
   CD66a, CD66a-Fragmente oder von CD66a abgeleitete Glykostrukturen, oder CD66a-Liganden, sowie DNA, die für CD66a. CD66a-Isoformen oder CD66a-Fragmente kodiert, die die Expression von CD66a oder CD66a-Ligand induziert,
   oder
(b) zur negativen Regulierung
   Antikörper, die spezifisch an eine oder mehrere der funktionellen Domänen von CD66a oder seiner Liganden binden, oder anti-sense Oligonukleotide oder anti-sense RNA, die die Expression von CD66a oder CD66a-Ligand hemmen.

Das Protein CD66a, das auch als biliäres Glykoprotein (BGP), "transmembrane carbonembryonic antigen" oder humanes C-CAM bezeichnet wird, ist ein spezielles Adhäsionsmolekül. Im folgenden wird die Bezeichnung CD66a verwendet. Das CD66a codierende Gen wurde bereits cloniert (Hinoda et al., PNAS 85, 6959-6963, 1988). Die Anmelder der vorliegenden Anmeldung beschrieben bereits 1991 den weltweit einzigen CD66a-spezifischen monoclonalen Antikörper (Drzeniek et al., Cancer Letters 56, 173-179 (1991); Stoffel et al., J. Immunol. 150, 4978-4984 (1993)). Dieser Antikörper wird als 4D1/C2 bezeichnet und wurde am 22. Oktober 1998 bei der DSMZ (Deutsche Sammlung von Mikroorganismen und Zellkulturen), Mascheroder Weg, Braunschweig unter der Hinterlegungsnummer DSM ACC2371 hinterlegt.

Überraschenderweise wurde nun gefunden, daß der Faktor CD66a in Tumorkapillaren exprimiert wird, während die Blutgefäße der entsprechenden Normalgewebe negativ sind.

In einem humanen Leydig-Zell-Tumor ließen sich die einzelnen Stadien der Gefäßneubildung genau verfolgen. Hierbei wurde gefunden, daß bestimmte Stadien der Gefäßneubildung mit dem Auftreten der folgenden Faktoren korreliert werden können:
1. Proliferation von Endothelzellen: VEGF (vascular endothelial growth factor), VEGF-Rezeptoren
2. Ausbildung von Gefäßlumina: CD66a
3. Nächster Differenzierungsschritt: Endostatin
4. Nächster Differenzierungsschritt: Angiostatin

Es konnte in jüngsten Versuchen der Erfinder mit Hühnerembryonen gezeigt werden, daß CD66a ein potenter angiogenetischer Faktor ist und die Gefäßneubildung bei Normal- und Tumorgewebe fördert.

Es konnte ebenfalls gezeigt werden, daß durch einen gegen CD66a gerichteten Antikörper die Blockierung von CD66a erfolgte und die Kapillarbildung, die für ein Tumorwachstum notwendig ist, gehemmt wird. Ein Tumorwachstum kann nicht mehr stattfinden.

CD66a läßt sich in menschlichen Tumoren in neugebildeten Blutgefäßen (Kapillaren) in einem definierten Differenzierungsfenster, nämlich im Stadium der Lumenbildung, nachweisen. In einem in-vitro Differenzierungsmodell hemmt ein monoclonaler CD66a-Antikörper die Ausbildung von Gefäßrohren (tube formation) durch humane Endothelzellen. Diese Ergebnisse belegen, daß CD66a eine wesentliche Rolle bei der Angiogenese spielt. Aus der Expression von CD66a in Tumorgefäßen und der Hemmung der Ausbildung kapillärer Strukturen in-vitro durch einen monoclonalen CD66a-Antikörper folgt, daß durch funktionelle Blockierung von CD66a die Tumorangiogenese gehemmt werden kann.

Versuche mit Transfektomen haben gezeigt, daß CD66a an sich selbst bindet (homotypische Bindung) und an andere Mitglieder der CD66-Familie bindet. Die Lokalisation von CD66a in neu gebildeten Endothelien am basalen Zellpol sowie die Hemmung der Kapillarbildung ist ein Indiz dafür, daß CD66a mit Bestandteilen der extrazellulären Matrix interagiert.

Für die einerseits erfindungsgemäß beabsichtigte Hemmung von CD66a sind insbesondere Antikörper, die spezifisch an eine oder mehrere der funktionellen Domänen von CD66a oder seiner Liganden binden, geeignet. Bevorzugt werden monoklonale Antikörper, die gegen adhäsive, funktionell bedeutsame Domänen von CD66a gerichtet sind, verwendet-. Ferner besitzt CD66a Glykostrukturen, die angiogenetisch wirksam sein können, so z.B. LewisX und Sialyl-LewisX-Gruppen. Bevorzugt wird der oben bereits erwähnte monoclonale CD66a-Antikörper 4D1/C2 verwendet. Dadurch kommt es zu einer Hemmung der Tumorangiogenese über eine funktionelle Inaktivierung von CD66a. Die funktionelle Inaktivierung von CD66a erfolgt dabei durch Hemmung der Wechselwirkung zwischen CD66a und möglichen Liganden. Hierbei werden Strukturen blockiert, die die Interaktion vermitteln. Ferner können auch lösliche Liganden bzw. lösliche Ligandendomänen eingesetzt werden, um die Interaktion zu blockieren. Die Erfindung betrifft auch die Verwendung rekombinanter Domänen, die einem CD66a-Fragment entsprechen, sowie Fragmente von Antikörpern, die im wesentlichen mit dem Epitop von CD66a reagieren. Dadurch wird die von CD66a ausgehende Signalkette blockiert. Die eingesetzten Verbindungen können auch in geeigneter Weise modifiziert werden, so daß sie z.B. irreversibel an den Rezeptor binden.

Für die andererseits erfindungsgemäß beabsichtigte Förderung der Angiogenese sind insbesondere CD66a als Gesamtmolekül, CD66a-Domänen sowie spezifische Glykostrukturen von CD66a geeignet. In diesen Fällen wird die lösliche Molekülform an Orten des Körpers appliziert, an denen eine Angiogenese ausgelöst werden soll (z.B. im Herzmuskel). Ferner kann auch eine DNA Verwendung finden, die für CD66a oder Teile des CD66a-Proteins kodiert. Die DNA kann auch in Vektoren integriert sein, die in der Gentherapie gebräuchlich sind (z.B. Adenoviren). Auch durch Gabe von einfacher Plasmid-DNA kann eine Synthese des Proteins erreicht werden. Die positive Beeinflussung der Angiogenese wird durch eine Förderung der Interaktionen zwischen CD66a und CD96a-Liganden bewirkt.

Verfahren zur Gewinnung oben erwähnter Antikörper, die zur Hemmung der Angiogenese eingesetzt werden können, sind dem Fachmann bekannt und umfassen beispielsweise bezüglich polyclonaler Antikörper die Verwendung von CD66a oder eines Fragments davon als Immunogen zur Immunisierung geeigneter Tiere und die Gewinnung von Serum. Verfahren zur Herstellung monoclonaler Antikörper sind dem Fachmann ebenfalls bekannt. Dazu werden beispielsweise Zell-Hybride aus Antikörper produzierenden Zellen und Knochenmark-Tumorzellen (Myelomzellen) hergestellt und cloniert. Anschließend wird ein Clon selektioniert, der einen Antikörper produziert, der für CD66a spezifisch ist. Dieser Antikörper wird dann gemäß Standardverfahren hergestellt. Beispiele von Zellen, die Antikörper produzieren, sind Milzzellen, Lymphknotenzellen, B-Lymphozyten etc.. Beispiele von Tieren, die zu diesem Zweck immunisiert werden können, sind Mäuse, Ratten, Pferde, Ziegen und Kaninchen. Die Myelomzellen lassen sich aus Mäusen, Ratten, Menschen oder anderen Quellen erhalten. Die Zellfusion kann man beispielsweise durch das allgemein bekannte Verfahren von Köhler und Milstein durchführen. Die durch Zellfusion erhaltenen Hybridome werden mittels CD66a nach dem Enzym-Antikörper-Verfahren oder nach einem ähnlichen Verfahren abgesucht. Clone werden beispielsweise mit dem Grenz-Verdünnungsverfahren erhalten. Die erhaltenen Clone werden BA-LB/c-Mäusen intraperitoneal implantiert, nach 10 bis 14 Tagen wird der Ascites der Maus entnommen, und der monoclonale Antikörper durch bekannte Verfahren (beispielsweise Ammoniumsulfatfraktionierung, PEG-Fraktionierung, Ionenaustauschchromatographie, Gelchromatographie oder Affinitätschromatographie) gereinigt. Der gewonnene Antikörper kann direkt verwendet werden oder es kann ein Fragment davon verwendet werden. In diesem Zusammenhang bedeutet der Begriff "Fragment" alle Teile des Antikörpers (z.B. Fab-, Fv- oder "single chain Fv"-Fragmente), welche die gleiche Epitopspezifität wie der vollständige Antikörper aufweisen.

In einer Ausführungsform ist der genannte monoclonale Antikörper ein aus einem Tier (z.B. Maus) stammender Antikörper, ein humanisierter Antikörper oder ein chimärer Antikörper oder ein Fragment davon. Chimäre, menschlichen Antikörper ähnelnde oder humanisierte Antikörper besitzen eine herabgesetzte potentielle Antigenität, jedoch ist ihre Affinität gegenüber dem Ziel nicht herabgesetzt. Die Herstellung von chimären und humanisierten Antikörpern bzw. von den menschlichen Antikörpern ähnelnden Antikörpern wurde ausführlich beschrieben (Noguchi, Nippon Rinsho, 1997, 55(6), S. 1543-1556; van Hogezand, Scand. J. Gastroenterol. Suppl. 1997, 223, S. 105-107). Humanisierte Immunglobuline weisen variable Grundgerüstbereiche auf, die im wesentlichen von einem humanen Immunglobulin stammen (mit der Bezeichnung Akzeptor-Immunglobulin) und die Komplementarität der determinierenden Bereiche, die im wesentlichen von einem nichtmenschlichen Immunglobulin (z.B. von der Maus) stammen (mit der Bezeichnung Donor-Immunglobulin). Die (der) konstante(n) Bereich (e) stammt/stammen, falls vorhanden, auch im wesentlichen von einem menschlichen Immunglobulin. Bei der Verabreichung an menschliche Patienten bieten die erfindungsgemäßen humanisierten (sowie die menschlichen) anti-CD66a-Antikörper eine Reihe von Vorteilen gegenüber Antikörpern von Mäusen oder anderen Spezies: (a) das menschliche Immunsystem sollte das Grundgerüst oder den konstanten Bereich des humanisierten Antikörpers nicht als fremd erkennen und daher sollte die Antikörper-Antwort gegen einen solchen injizierten Antikörper geringer ausfallen als gegen einen vollständig fremden Maus-Antikörper oder einen partiell fremden chimären Antikörper; (b) da der Effektorbereich des humanisierten Antikörpers menschlich ist, dürfte er mit anderen Teilen des menschlichen Immunsystems besser interagieren, und (c) injizierte humanisierte Antikörper weisen eine Halbwertszeit auf, die im wesentlichen zu der von natürlich vorkommenden menschlichen Antikörpern äquivalent ist, was es erlaubt, kleinere und weniger häufige Dosen im Vergleich zu Antikörpern anderer Spezies zu verabreichen.

Die vorstehend beschriebene konventionelle Technologie kann auch durch die Verwendung rekombinatorischer Phagen-Libraries ergänzt oder ersetzt werden (Felici et al., Biotechnol. Rev. 1, S. 149-183 (1995); Hoogenboom et al., Immunotechnology 4, S. 1-20 (1998)). Rekombinante Phagenlibraries können in den Antigen-bindenden Regionen der von Phagen präsentierten Antikörperfragmente zufällige Peptidstrukturen aufweisen. Der Vorteil dieser Technologie liegt u.a. darin, daß in klonierten Phagen die Information über die Aminosäuresequenz der Antigen-Bindungsstrukturen unmittelbar vorliegt.

Die Domänen von CD66a bzw. der CD66a-Liganden, deren Blockierung eine funktionelle Inaktivierung von CD66a bewirkt, können in beliebiger Weise rekombiniert werden und in Moleküle eingebaut, die zu therapeutischen Zwecken geeignet sind, verwendet werden (z.B. zur Erreichung besserer immunologischer

Verträglichkeit). Die reaktiven Domänen können auch gemäß molekularbiologischer Standardmethoden, z.B. bakteriell oder in Insektenzellen, exprimiert werden.

Vorzugsweise kann die Hemmung der Interaktion zwischen CD66a und potentiellen Liganden auf folgenden Wegen erfolgen (negative Regulierung):
- Hemmung durch Antikörper und -fragmente gegen die funktionelle Domäne von CD66a
- Hemmung durch Antikörper und -fragmente gegen die funktionellen Domänen der CD66a-Liganden
- Hemmung durch die funktionelle Domäne von CD66a
- Hemmung durch die funktionelle Domäne der CD66a-Liganden
- Hemmung der endogenen Bildung von CD66a oder CD66a-Liganden durch Einsatz von Anti-Sense-Oligonukleotiden.

Vorzugsweise kann die Förderung der Interaktion zwischen CD66a und potentiellen Liganden auf folgenden Wegen erfolgen (positive Regulierung):
- Applikation des nativen, mittels biochemischer Methoden gereinigten Moleküls
- Applikation rekombinanter CD66a-Fragmente
- Applikation angiogenetisch aktiver, aus CD66a isolierter Glykostrukturen
- Applikation vollsynthetisch oder teilsynthetisch hergestellter Glykostrukturen, deren Struktur aus angiogenetisch aktiven Glykostrukturen von CD66a abgeleitet wurde,
- Applikation einer DNA, die für das vollständige CD66a-Protein davon kodiert, in Form von geeigneten Vektoren oder Plasmiden
- Applikation einer DNA, die für Isoformen oder Fragmente von CD66a kodiert, in Form von geeigneten Vektoren oder Plasmiden.

Die erfindungsgemäßen pharmazeutischen Zusammensetzungen können auf jedem beliebigen Weg verabreicht werden, der geeignet ist, das beabsichtigte Gewebe zu erreichen. Bevorzugt erfolgt die Verabreichung parenteral, bevorzugt oral, intravenös oder intratumoral. Zur Verabreichung wird die Substanz in einer für die jeweilige Verabreichungsart geeigneten Formulierung unter Zuhilfenahme entsprechender üblicher pharmazeutischer Exzipientien verwendet. Die Entwicklung oral applizierbarer Pharmaka erfolgt auf zwei Wegen. Zum einen kann die Interaktion zwischen Ligand und Rezeptor z.B. durch Röntgenstrukturanalyse oder NMR-Spektroskopie modelliert werden. Zum anderen können chemische kombinatorische Libraries (Myers, Current Opinion in Biotechnology 8, S. 701-717 (1997) verwendet werden. Hier wird die Interaktion des Liganden bzw. Rezeptors mit zunächst weitgehend zufällig zusammengestellten chemischen Verbindungen untersucht. Wenn eine Bindung nachgewiesen wurde, lassen sich die Bindungseigenschaften durch Auswahl ähnlicher Verbindungen näher eingrenzen.

Die Dosierung und die Posologie der Verabreichung der erfindungsgemäßen Verbindungen wird vom Arzt anhand der patientenspezifischen Parameter wie z.B. Alter, Gewicht, Geschlecht, Schwere der Erkrankung, etc. bestimmt.

Entsprechend der Art der Verabreichung wird das Medikament in geeigneter Weise formuliert, z.B. in Form von Lösungen, Suspensionen, als Pulver, Tablette oder Kapsel oder Injektionspräparate, die nach üblichen galenischen Verfahren hergestellt werden.

Die Infusions- oder Injektionslösungen sind bevorzugt wäßrige Lösungen oder Suspensionen, wobei es möglich ist, diese vor Gebrauch herzustellen, beispielsweise aus lyophilisierten Präparaten, die den Wirkstoff alleine oder zusammen mit einem Träger, wie Mannit, Lactose, Glucose, Albumin und dergleichen, enthalten. Die gebrauchsfertigen Lösungen werden sterilisiert und gegebenenfalls mit Hilfsmitteln vermischt, beispielsweise mit Konservierungsstoffen, Stabilisatoren, Emulgatoren, Lösungsvermittlern, Puffern und/oder Salzen zur Regulierung des osmotischen Drucks. Die Sterilisierung kann durch Sterilfiltration durch Filter mit einer kleinen Porengröße erzielt werden, wonach die Zusammensetzung gegebenenfalls lyophilisiert werden kann. Antibiotika können auch zugesetzt werden, um die Beibehaltung der Sterilität zu unterstützen.

Die pharmazeutischen Zusammensetzungen enthalten eine therapeutisch wirksame Menge einer oder mehrere oben angegebener Wirksubstanz(en) zusammen mit üblichen Hilfs- und Trägerstoffen. Diese sind bevorzugt organische oder anorganische flüssige pharmazeutisch verträgliche Träger, die für die beabsichtigte Verabreichung geeignet sind, und die mit den aktiven Inhaltsstoffen nicht nachteilig wechselwirken.

Die erfindungsgemäßen pharmazeutischen Präparate werden in Dosiseinheitsformen abgegeben, beispielsweise als Ampullen.

Die Erfindung betrifft auch ein Verfahren zur Produktion einer pharmazeutischen Zusammensetzung, die dadurch gekennzeichnet ist, daß die erfindungsgemäße Verbindung mit einem pharmazeutisch verträglichen Träger vermischt wird.

"Substanzen, die die Expression von CD66a oder CD66a-Ligand hemmen" werden bevorzugt auf gentherapeutischem Weg verbreicht, indem in Tumorzellen beispielsweise anti-sense Oligonukleotide zu CD66a und/oder CD66a-Ligand eingebracht werden. Diese Oligonukleotide sind von den bekannten Sequenzen für CD66a oder CD66a-Ligand abgeleitet (Hinoda et al., Proc. Natl. Acad. Sci USA 85, S. 6959 (1988)). Die anti-sense Oligonukleotide können auch die Größe einer RNA ereichen, die zu Bereichen der mRNA des Gens komplementär ist und an diese bindet. Es entsteht dann ein Duplexmolekül, das der Translation der mRNA entzogen ist. Damit kann eine Hemmung der Genexpression erreicht werden. Der Ausdruck "anti-sense-Oligonukleotid" umfaßt jegliches DNA- oder RNA-Molekül, das komplementär zu Bereichen der CD66a-RNA oder CD66a-Ligand-RNA, insbesondere mRNA und ganz besonders Regulationselementen dieser, ist und durch Bindung an diese Bereiche eine Hemmung der Genexpression bewirkt. Die anti-sense-Oligonukleotide können als solche oder, wenn sie länger sind, in Form eines sie kodierenden Vektors bzw. Vektorkonstrukts, das gelegentlich auch als "Minigen" bezeichnet wird, vorliegen. Ein solcher Vektor kann ein üblicher Expressionsvektor sein. Günstig kann es sein, wenn die Expression der für sie kodierenden Sequenz unter der Kontrolle eines konstitutiven oder induzierbaren Promotors, wie eines Gewebe- oder Tumorspezifischen Promotors, steht. Die Einbringung der anti-sense Moleküle kann durch übliche Verfahren erfolgen. Liegen die anti-sense-Oligonukleotide als solche oder in Form eines sie kodierenden Vektors vor, eignen sich z.B. Transfektionstechniken oder es eignet sich z.B. eine Verpackung in Liposomen.

"Substanzen, die die Expression von CD66a oder CD66a-Ligand induzieren", sind beispielsweise DNA-Moleküle, die für CD66a bzw. angiogenetisch wirksame CD66a-Fragmente, oder aber für CD66a-Liganden bzw. angiogenetisch wirksame Liganden-Fragmente kodieren. Die Expression wird durch geeignete regulatorische Sequenzen gesteuert. Die Verabreichung der DNA erfolgt nach Protokollen, die einem Fachmann der Gentherapie bekannt sind. So kommt z.B. eine Verpackung der DNA in Viruspartikel (z.B. Adenoviren), oder aber die Gabe von nackter Plasmid-DNA in Frage.

Erfindungsgemäß kann mit der Angiogenese hemmenden pharmazeutischen Zusammensetzung das Wachstum aller soliden Tumore des Körpers gehemmt werden. Beispiele sind epitheliale Tumore (z.B. Platten-, Zylinder-, Drüsen-, Übergangsepithel), mesenchymale Tumore (z.B. Faser, Muskel, Knorpel und Kochengewebe), Mischtumoren (gemischt epithelial, gemischt mesenchymal, epithelial-mesenchymal), Tumore der blutbildenden und lymphatischen Gewebe (Knochenmark, lymphatische Gewebe), Tumoren der serösen Höhlen (z.B. Lungenfell, Herzbeutel, Bauchfell, Gelenkinnenhaut), Tumore des Nervensystem (z.B. Ganglienzellen, Neuroepithel, Neroglia, Hirnhäute, Sympathikus, periphere Nerven), Tumore des Magen-Darm-Trakts und Tumore einzelner Organe. Bevorzugt wird erfindungsgemäß das Wachstum von Tumoren der Bronchien und der Lunge, der Brust, der Leber, der Galle, der Pankreas, der Nieren und Harnorgane, des Magens, des Dickdarms, des Mastdarms, der Prostata und der Gebärmutter gehemmt.

Erfindungsgemäß kann mit der Angiogenese-fördernden pharmazeutischen Zusammensetzung die Gefäßneubildung bei solchen Erkrankungen induziert werden, bei denen der krankheitsbedingte Verschluß von Gefäßen zu einer Minderversorgung des Gewebes mit Sauerstoff und Nährstoffen führt. Als Beispiele seien koronare Herzerkrankungen oder Minderdurchblutungen der Extremitäten bei Diabetikern, starken Rauchern oder Patienten mit hohem Blutdruck genannt.

Die Erfindung wird anhand der Figuren näher beschrieben:
- Fig. 1: Lokalisation von CD66a in den Gefäßen eines humanen Leydig-Zell-Tumors. Die immunhistochemische Färbung wurde mit dem Antikörper 4D1/C2 durchgeführt.
Fig. 1a: eine der angefärbten Tumorkapillaren ist mit einem Pfeil markiert (x350)
Fig. 1b: Vergrößerung eines Bereichs aus Fig. 1a. Der Pfeil weist auf die Färbung einer Endothelzelle hin (x950)
- Fig. 2: Chemotaktischer Effekt von CD66a (=BGP) auf HDMEC
- Fig. 3: Proliferation von HDMEC nach der Stimulation mit CD66a (= BGP)
- Fig. 4: Wirkung von CD66a auf die Ausbildung kapillarähnlicher Gefäßrohre in Zellkultur

Die Erfindung wird anhand der folgenden Beispiele näher erläutert.

### Beispiel 1

### Lokalisation von CD66a in Tumorkapillaren

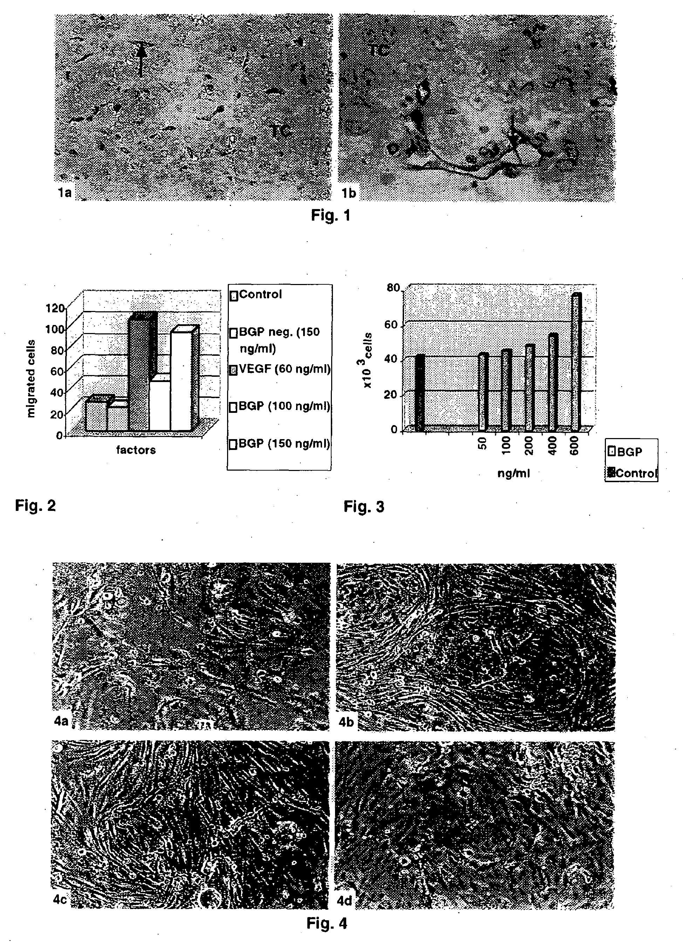

Unter Verwendung des monoclonalen Anti-CD66a-Antikörpers 4D1/C2 wurden Tumoren immunhistochemisch gefärbt und lichtmikroskopisch untersucht. Hierbei wurde zusätzlich zu den zuvor angewendeten immunhistochemischen Verfahren (Prall et al. (1996), J. Histochem. Cytochem. 44, 35-41) eine Verstärkungsmethode mit Nickel und Glukoseoxidase eingesetzt. Weiterhin wurden nach immunhistochemischer Färbung mit dem monoclonalen Antikörper 4D1/C2 elektronenmikroskopische Analysen durchgeführt (s. Fig. 1).

Es wurden menschliche Hodentumoren, Hirntumoren sowie Prostata-, Harnblasen- und Nierenkarzinome immunhistochemisch untersucht. CD66a wurde in Endothelzellen und in der Basalmembran der Tumorkapillaren lokalisiert. Ausgereifte, nicht-prolieferierende ruhende Gefäße der untersuchten Organe waren negativ. Wird der Tumor nach funktionellen Gesichtspunkten in unterschiedliche zonale Abschnitte, nämlich Tumorzellen, Tumorrand und Tumorumgebung unterteilt, dann ist die positive Immunreaktion in den neuformierten Tumorkapillaren am Tumorrand zu finden. Dies deutet auf eine Funktion von CD66a in sehr frühen Phasen der Gefäßneubildung (Neoangiogenese) hin.

### Beispiel 2

### Wirkung von CD66a auf die Proliferation und Chemotaxe kultivierter Endothelzellen

Um die Wirkung von CD66a auf die Proliferation und Chemotaxe kultivierter Endothelzellen zu testen, wurde das Glykoprotein aus Membranfaktionen humaner Granulozyten gereinigt: Die Isolierung der Membranfraktion folgte etablierten Methoden (Drzeniek et al. (1991), Cancer Letters 56, 173-179; Stoffel et al. (1993), J. Immunol. 150, 4978-4984). Nach Extraktion der Membranglykoproteine mit einem nichtionischen Detergens wurden diese an einen immobilisierten monoclonalen CD66-Antikörper gebunden und mit Glycin-HCl bei pH 2,2 eluiert. Das Eluat wurde nach Neutralisierung gelchromatographisch über Superdex 200 (Pharmacia) weiter getrennt. Die CD66a-positiven Fraktionen wurden gepoolt. Verunreinigungen im niedermolekularen Bereich wurden mittels Ultrafiltration unter Verwendung eines Filters mit einem Ausschluß von 100 kD abgetrennt. Mittels SDS-PAGE im Silbergel wurde in Verbindung mit einem Western-Blot gezeigt, daß der Überstand ausschließlich CD66a enthielt. Diese Fraktion wurde für Zellkulturversuche mit Endothelzellen verwandt.

Die Versuche wurden mit zwei verschiedenen humanen Endothelzellformen durchgeführt, nämlich mit HUVEC-(human umbelical vein endothelial cells) und HDMEC-(human dermal microvascular endothelial cells) Zellen.

Die Wirkung von CD66a auf die Proliferation wurde in Monolayerkultur überprüft. Endothelzellen wurden in einer definierten Zahl auf eine Mikrotiterplatte ausgesät. Nach 72 Stunden wurde die Zahl der Endothelzellen in stimulierten und nicht-stimulierten Kulturen verglichen. Es zeigte sich, daß CD66a die Proliferation beider Zellinien in dosisabhängiger Weise stimulierte.

Die Wirkung von CD66a auf die Chemotaxe wurde in einem zweikammrigen Kultursystem (sog. Boyden-Chamber) untersucht. In der oberen Kammer werden die Zellen kultiviert, die untere Kammer enthält chemotaktische Substanzen. Die beiden Kammern sind durch ein Polycarbonatfilter getrennt, das einen Durchtritt der Zellen gestattet. Nach Zugabe von CD66a in die untere Kammer zeigte sich ein dosisabhängiger chemotaktischer Effekt auf beide Endothelzellinien. Die Wirkung von CD66a war mit der Wirkung von VEGF vergleichbar. Wie aus Fig. 2 ersichtlich, besitzt CD66a (=BGP) ab einer Konzentration von 100 ng/ml einen chemotaktischen Effekt. Bei einer Konzentration von 150 ng/ml ist die chemotaktische Wirkung nur geringfügig geringer als die von VEGF (vascular endothelial growth factor).

Der chemotaktische Effekt von CD66a wurde auch in Kombination mit VEGF und bFGF ("basic fibroblast growth factor") analysiert. Der chemotaktische Effekt von VEGF bzw. bFGF wurde durch CD66a jeweils um etwa 30% gesteeigert.

Kultivierte humane mikrovaskuläre Hautfibroblasten wurden mit CD66a (=BGP) in Konzentrationen von 50, 100, 200, 400 und 600 ng/ml inkubiert. Ab einer Konzentration von 200 ng/ml war eine proliferationssteigernde Wirkung von CD66a nachweisbar. Dies ist in Fig. 3 gezeigt.

Mit der positiven Wirkung auf Proliferation und Chemotaxe erfüllt CD66a die Hauptkriterien von Angiogenesefaktoren.

### Beispiel 3

Wirkung von CD66a auf die Ausbildung kapillarähnlicher Gefäßrohre in Zellkultur.

Die in Beispiel 2 beschriebenen Versuchsergebnisse legen die Annahme nahe, daß CD66a kausal an der Ausbildung neuer Gefäße (Neoangiogenese) beteiligt ist. Um diese Hypothese zu testen, wären Tierversuche am geeignetsten. Da es sich bei CD66a jedoch um ein humanes Glykoprotein handelt, ist damit zu rechnen, daß die Wirkung im Versuchstier aufgrund der Speziesunterschiede nicht oder nur gering ausgeprägt ist. Für diese Annahme spricht der Befund, daß der monoclonale anti-CD66a-Antikörper 4D1/C2 in menschlichen Geweben eine gute Reaktion zeigt. In den entsprechenden Geweben von Ratte und Maus ist die Reaktion schwach und nur schwer von einer unspezifischen Hintergrundreaktion zu unterscheiden. Der Antikörper 4D1/C2 bindet anscheinend an eine antigene Struktur, die in dieser Form in Nagern nicht vorkommt.

Um die durch Speziesunterschiede bedingten Probleme zu umgehen, werden Zellkulturmodelle verwendet, in denen Endothelzellen unter bestimmten Bedingungen zu Gefäßrohren auswachsen, die neugebildeten Kapillaren entsprechen (engl.: "tube formation"). Hierzu werden die Zellen in Anwesenheit von spezifischen Wachstumsfaktoren wie z.B. VEGF ("vascular endothelial growth factor") oder FGF-2 ("fibroblast growth factor") in einer bindegewebigen Matrix kultiviert. Diese Kulturform stellt eine gute Annäherung an in-vivo-Bedingungen dar.

Um die Bedeutung von CD66a für die Kapillarbildung zu untersuchen, wurden HUVEC- und HDMEC-Zellen in dreidimensionalen Collagen-I-Gelen gezüchtet. In Anwesenheit von Wachstumsfaktoren wie VEGF und FGF-2 bilden die Endothelzellen tubuläre Strukturen aus, die neugebildeten Kapillaren entsprechen. In Anwesenheit des monoclonalen CD66a-Antikörpers 4D1/C2 wurde die Ausbildung von Gefäßrohren gehemmt. Ein zweiter monoclonaler Antikörper, der gegen ein anderes Epitop auf CD66a gerichtet ist, hatte keinen Effekt auf die Entstehung von Tubuli. Diese Versuche belegen einen funktionellen Zusammenhang zwischen der Expression von CD66a und der Neubildung von kapillarähnlichen Gefäßrohren. Mit Hilfe des Antikörpers wird ferner die funktionelle Domäne von CD66a definiert.

Die Ergebnisse obiger Versuche sind in Fig. 4 gezeigt:

In Anwesenheit des Angiogenesefaktors VEGF (50 ng/ml) bilden sich kapillarähnliche Strukturen aus (s. Abb 4a). Kapillarähnliche Strukturen äußern sich in Strängen ("tubes"), in denen die länglich ausgezogenen Endothelzellen parallel angeordnet sind. Diese Stränge sind Fischzügen vergleichbar. In der Mitte von Fig. 4a liegt eine Region, in der die Endothelzellen abgerundet sind. Dies sind keine "tubes":

In Fig. 4b ist das Ergebnis eines Experiments dargestellt, in dem die Kapillarbildung in Anwesenheit von VEGF (50 ng/ml) und CD66a (150 ng/ml) untersucht wurde. Im Vergleich mit Fig. 4a sind fast alle Enodothelzellen an der Ausbildung von "tubes" beteiligt. Außerdem ist ein Verzweigungsmuster erkennbar, das für die weitere Differenzierung des Angiogeneseprozesses spricht. CD66a verstärkt somit den angiogenetischen Effekt von VEGF.

In Fig. 4c wurden die Endothelzellen in Anwesenheit von CD66a (300 ng/ml) und in Abwesenheit von VEGF kultiviert. Es zeigen sich kapillarähnliche Strukturen.

In Fig. 4d ist das Ergebnis eines Experiments dargestellt, in dem die Endothelzellen in Anwesenheit des monoklonalen Antikörpers 4D1/C2 kultiviert wurden. Die Bildung von Kapillaren ist vollständig gehemmt. Aus diesem Experiment folgt, daß der Antikörper 4D1/C2 an eine Domäne von CD66a bindet, die für die Kapillarbildung essentiell ist.

## Patentansprüche

1. Verwendung von einem oder mehreren Stoffen zur Herstellung einer pharmazeutischen Zusammensetzung zur Beeinflussung der Angiogenese, wobei die Stoffe umfassen:
(a) zur positiven Regulierung
CD66a, CD66a-Fragmente oder von CD66a abgeleitete Glykostrukturen, oder CD66a-Liganden, sowie DNA kodierend für CD66a, CD66a-Isoformen oder CD66a-Fragmente, die die Expression von CD66a oder CD66a-Ligand induziert
oder
(b) zur negativen Regulierung
Antikörper, die spezifisch an eine oder mehrere der funktionellen Domänen von CD66a oder seiner Liganden binden, oder anti-sense Oligonukleotide oder anti-sense RNA, die die Expression von CD66a oder CD66a-Ligand hemmen.

2. Verwendung nach Anspruch 1(b), **dadurch gekennzeichnet, dass** der Antikörper ein Anti-CD66a-Antikörper ist.

3. Verwendung nach Anspruch 2, **dadurch gekennzeichnet, dass** der Antikörper der monoklonale Anti-CD66a Antikörper 4D1/C2 ist, der bei der DSMZ Braunschweig am 22. Oktober 1998 unter DSM ACC2371 hinterlegt wurde.

4. Verwendung nach einem der Ansprüche 1(b) bis 3, **dadurch gekennzeichnet, dass** sie zur Unterbrechung der Tumorangiogenese von Lungen-, Brust- und Dickdarmkrebs befähigt ist.

## Claims

1. Use of one of more substances for the production of a pharmaceutical composition for influencing angiogenesis, the substances comprising:
(a) for positive regulation
CD66a, CD66a fragments of glycostructures derived from CD66a or CD66a ligands as well as DNA coding for CD66a, CD66a isoforms or CD66a fragments which induces the expression of CD66a or CD66a ligand
or
(b) for negative regulation
antibodies specifically binding to one or more functional domains of CD66a or its ligands or anti-sense oligonucleotides or anti-sense RNA inhibiting the expression of CD66a or CD66a ligand.

2. Use according to claim 1(b), **characterized in that** the antibody is an anti-CD66a antibody.

3. Use according to claim 2, **characterized in that** the antibody is the monoclonal anti-CD66a antibody 4D1/C2 which was deposited with DSMZ (German-Type Collection of Microorganisms and Cell Cultures) Braunschweig under DSM ACC2371.

4. Use according to any of claims 1(b) to 3, **characterized in that** it is capable of stopping the tumor angiogenesis of lung, breast and colon cancer.

## Revendications

1. Utilisation d'un ou plusieurs composés pour la préparation d'une composition pharmaceutique pour influencer l'angiogenèse, **caractérisée en ce que** les composés comprennent :
- pour une régulation positive
CD66a, fragments de CD66A ou bien des glycostructures conduisant à CD66a, ou bien des ligands CD66a, ainsi que l'ADN codant pour CD66a, les formes iso CD66a ou bien des fragments de CD66a, qui induisent l'expression de CD66a ou du ligand CD66a
- pour une régulation négative
les anticorps qui se lient spécifiquement à une ou plusieurs domaines fonctionnels de CD66a ou à ses ligands, ou bien les oligonucléotides anti-sens ou bien l'ARN anti-sens, qui préviennent l'expression de CD66a ou bien du ligand CD66a.

2. Utilisation selon la revendication 1, **caractérisée en ce que** l'anticorps comprend l'anticorps anti-CD66a.

3. Utilisation selon la revendication 2, **caractérisée en ce que** l'anticorps est l'anticorps 4D1/C2 monoclonal de l'anti-CD66a, qui a été déposé chez DSMZ Braunschweig le 22 octobre 1998 sous la désignation DSM ACC2371.

4. Utilisation selon l'une des revendications 1 à 3 **caractérisée en ce qu'**elle est capable d'interrompre l'angiogenèse tumorale du cancer de la langue, du sein et du gros intestin.
